# EUROPEAN PATENT APPLICATION

(11) **EP 0 528 573 A1**
(43) Date of publication of application: **24.02.1993**
(21) Application number: 92307050.2
(22) Date of filing: 03.08.1992
(51) Int. Cl.: A61B 17/56, F16B 13/12

(54) **Fastener for securing an orthopaedic device to a bone**

(30) Priority: 08.08.1991 GB 9117146
(71) Applicant: HOWMEDICA INTERNATIONAL INC., Shannon Co. Clare (IE)
(72) Inventor: Ashby, Alan Miles, Maidenhead, Berkshire, SL6 4LD (GB)
(74) Representative: Bridge-Butler, Alan James

(57) **Abstract**

A fastener for securing an orthopaedic device to a bone comprising an expandable plug made from a biodegradable or biostable material and having two segments (1,2) which when placed together provide an open ended tapered inner bore provided with retention means (120), and a retention element which can act on the orthopaedic device to locate it against said bone and which when entered into the bore under pressure locates in the retention means and acts to expand the plug, said segments being interconnected at their ends spaced away from the open end of the bore.

## Description

This invention relates to a fastener for securing an orthopaedic device to a bone and more particularly to a biodegradable multi-part fastener device.

According to the present invention a fastener for securing an orthopaedic device to a bone comprises an expandable plug made from a biodegradable or biostable material and having two or more segments which when placed together provide an open ended tapered inner bore provided with retention means, and a retention element which can act on the orthopaedic device to locate it against said bone and which when entered into the bore under pressure locates in the retention means and acts to expand the plug, and interconnection means spaced away from the open end of the inner bore for holding said segments in alignment during insertion.

In a preferred construction said interconnection between the segments is in the form of a hinge.

The segments may have outwardly projecting abutments to engage and locate in an opening in said bone to which the device is to be fastened.

The segments can be made of a biocompatable material which may be from the known family and classes of synthetic biodegradable materials such as polyglycolic, polylactic acid polymers and copolymers, polyhydroxybutyrate, polydioxanone etc., or from soluble type synthetic materials i.e., polyethylene oxide block copolymers, polyvinyl alcohols, cellulosics, etc., or from natural resorbable materials, such as fibrin, collagen, gelatin, dextran etc. Alternatively the segments may be constructed from an essentially biostable material such as a polyethylene, polypropylene or another synthetic polymer. Any of these materials may be of generally homogeneous form or reinforced by fillers or fibres of other or similar materials.

The outwardly projecting abutments can have an external tooth form of relatively large pitch and may be formed by radial and helical fins.

Alternative helical fins can be opposite handed so that when engaging bone these do not allow for any rotational unlocking of the fastener.

The retention means in the bore can be provided by a series of engagement ribs and these may be in the form of a tapered screw thread.

Preferably the retention element is a screw or a circumferentially ribbed pin and the screw or pin may be of generally cylindrical shape with an overlaying tapering tooth form or ribbing.

The retention means can be metallic from the range of metals used in orthopaedic implants, that is Cobalt-Chrome, Ti alloys, stainless steels or it can be made from synthetic polymer or polymeric, from the same range of biodegradable materials as are used in the segment construction or from some other synthetic polymer of known biocompatability.

Means can also be included for preventing expansion of the plug adjacent the open end of the bore, these means being in the form of a sleeve surrounding the plug.

The segments can be provided with guides to assist assembly together to form the expandable plug.

The invention can be performed in various ways but one embodiment will now be described by way of example and with reference to the accompanying drawings in which :-
Figure 1 is a side elevation of an assembled expandable plug for use in the fastener according to the present invention;
Figure 2 is an isometric view of the expandable plug in an open position and ready for use;
Figure 3 is an isometric view of the plug shown in Figure 2 in a partially closed position together with a sleeve which forms part of the wide end of the plug when assembled;
Figure 4 is a cross-sectional side view of the sleeve shown in Figure 3;
Figure 5 is a side elevation of a retention element in the form of a circumferentially ribbed pin for use with the expandable plug shown in Figures 1 to 4;
Figures 6, 7 and 8 show various stages in the assembly of the device when used to secure an orthopaedic device to a bone.

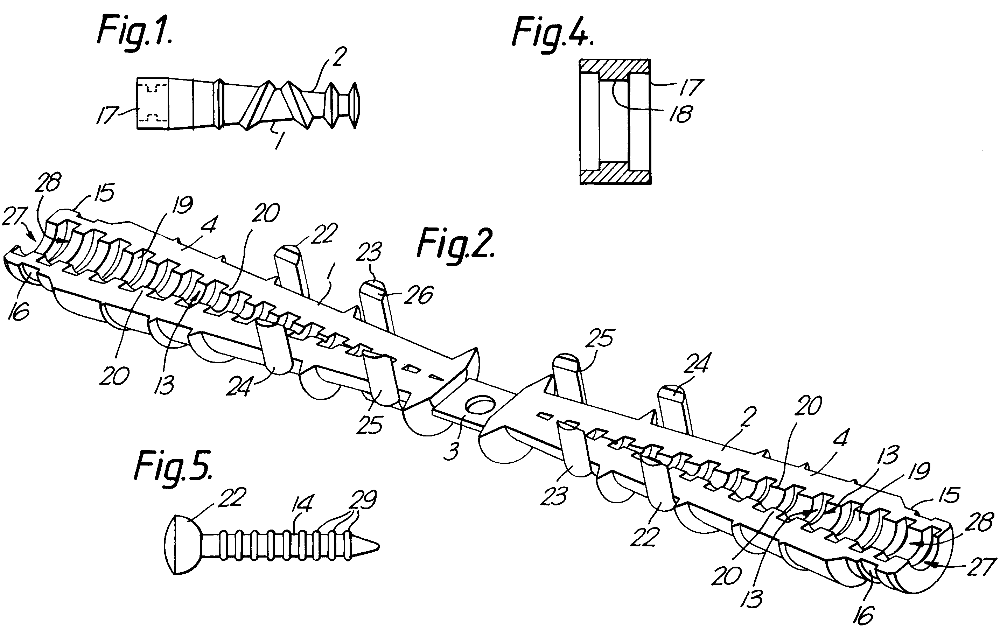

As shown in Figures 1, 2 and 3 an expandable plug which forms part of the fastener comprises a moulding have a first segment 1 and a second segment 2. The segments can be made of a biocompatable material which may be from the known family and classes of synthetic biodegradable materials such as polyglycolic, polylactic acid polymers and copolymers, polyhydroxybutyrate, polydioxanone etc., or from soluble type synthetic materials i.e., polyethylene oxide block copolymers, polyvinyl alcohols, cellulosics, etc., or from natural resorbable materials, such as fibrin, collagen, gelatin, dextran etc. Alternatively the segments may be constructed from an essentially biostable material such as a polyethylene, polypropylene or another synthetic polymer. Any of these materials may be of generally homogeneous form or reinforced by fillers or fibres of other or similar materials.

In the construction shown in the drawings the two segments are made as a single moulding being joined together by a web 3. Each segment is of substantially semicircular cross section so that when the two segments 1 and 2 are folded over around the web 3, which acts as a hinge in the manner shown in Figure 3 they can be placed together with their flat surfaces 4 in engagement to provide what is in effect a tapering cylindrical plug as shown in Figure 1, the web 3 acting as interconnecting means to hold the segments in alignment. In Figure 1 only the basic shape of the plug is shown, the details being apparent from Figures 2 and 3.

Outwardly projecting abutments are provided on the outer surfaces of the segments 1 and 2 and are in the form of longitudinally extending fins 5, 6 and 7. The fins are of substantially triangular cross-section and are of increasing height throughout the tapering length of the segment concerned. Thus, it will be seen that the fins 7 are higher than the fins 5 which are themselves of tapering shape. The depth of these fins is such that from one end of the segment to the other they provide a substantially straight line which is in alignment with an imaginary line defining a cylindrical shape around the tapering surfaces of the segments.

Further abutments are provided in the form of fins 8 to 12 of substantially triangular form and which once again are of increasing height throughout the tapering length of each segment. Thus, circumferentially extending fins 8 and 9 extend around the longitudinally extending axis of the assembled plug at right angles thereto, the fin 9 being higher than the fin 8. These two fins are followed by a further two fins 10 and 11 which are also of triangular cross-section but which are arranged as axially elongate rings. Thus, as will most clearly be seen from Figure 1 these rings are not normal to the longitudinally extending axis of the plug. If desired these rings could be replaced by fins which were helical. In any case, whether helical or axially elongate, the fins 10 and 11 are opposite handed, again as will be seen from Figure 1 and the fin 11 is higher than the fin 10. Finally the end of each is defined by a further radially extending fin or flange 12.

As mentioned above these fins or flanges 5 to 12 are all arranged so that their outer extremities are defined by an imaginary cylinder co-axial with the longitudinally extending centre line of the plug when the two segments are closed together.

The end of each segment spaced away from the web 3, which acts as a hinge, includes a neck portion 15 of reduced diameter which has a circumferential location groove 16 formed therein. When the two segments are folded together the two neck portions provide a cylindrical boss on which is placed a sleeve 17 provided with an outwardly projecting flange 18 which locates in the groove 16. This sleeve 17 when in position on the segments acts to locate the two segments together and to prevent expansion of the plug adjacent the open end 37 of a longitudinally extending bore 13 which is formed by the two segments the other end of the plug is held in alignment by the hinge 3. This bore 13 is provided by semicircular grooves 19 provided on the flat faces 4 of the segments. The outside diameter of the sleeve 17 is such that it is approximately equal to the longest diameter on the body of the assembled plug. The grooves 19 carry a series of circumferential ribs 20 which can be shaped so that when the segments are close together they form a tapered screw thread. Alternatively they may just provide a series of circumferentially extending ribs around the bore provided by the two grooves 19 when closed together.

In order to assist location of the segments when folded together guides 22, 23, 24 and 25 are provided on each segment. These guides consist of upwardly projecting arms, the outer ends of which are chamfered as indicated at 26, on guide 23 in Figure 2. These guides are effective when a retention device, to be described later, is inserted into the plug to expand it, in that they prevent a relative rotation of the two segments of the expanding plug. The guides 22, 23, 24 and 25 are not shown in Figure 1 in order to simplify this Figure.

As referred to above the bore 13 provided by the grooves 19 is tapered but at its outer open end indicated by reference numeral 27 in Figure 3 the taper ceases so there is an end portion which has walls which are substantially cylindrical, this portion being generally indicated by reference numeral 28. As will be seen this parallel sided portion is co-axial with the neck portion 15. This parallel portion 28 allows appropriate engagement of a screw into the internal screw thread when provided, or the insertion of the ribbed pin, to be described, and avoids expansion of this portion 28 on further insertion of the screw or pin.

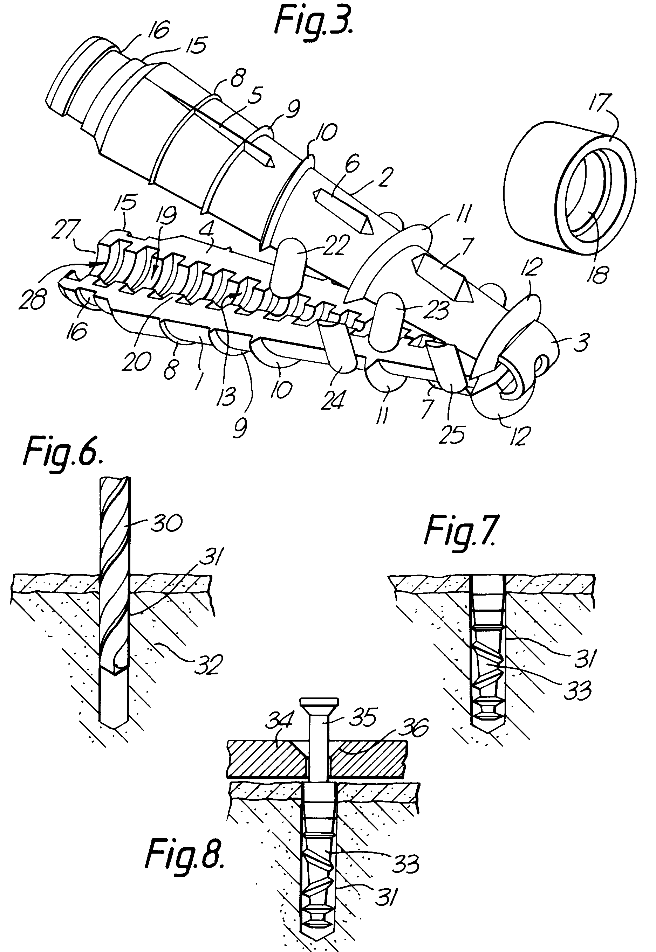

A retention element is provided which can be in the form of a screw or a circumferentially ribbed pin and the screw or pin may be of generally cylindrical shape with an overlying tapering toothed form or ribbing. This screw can be of normal configuration, for example as shown in Figure 8, and will not therefore be described further but a circumferentially ribbed pin for use with the plug is shown in Figure 5. The pin comprises a shank 14 having an enlarged head and provided with a series of circumferential ribs 29.

When the plug is used the retention element which is to be used to hold an orthopaedic device in place, in the manner to be described hereunder, is driven into the open end of the bore 27 and if there is a screw thread is rotated so that it extends into the bore. As the diameter of the bore decreases the retention element causes the segments 1 and 2 to move apart thus expanding the cross-sectional area of the plug. The ribs in the bore act to hold the retention means in place.

If the retention means is a pin as shown in Figure 5 which is not screwed however but merely ribbed then it can be driven in appropriately to have the same effect.

The retention element can be metallic and constructed from the range of metals used in orthopaedic implants, that is Cobalt-Chrome, Ti alloys, stainless steels or it can be made from synthetic polymer or polymeric, from the same range of biodegradable materials as are used in the segment construction or from some other synthetic polymer of known biocompatability.

The operative use of the fastener is shown diagrammatically in Figures 4, 5 and 6. In these arrangements a drill 30 is used to form a hole or opening 31 in the bone 32 to which an orthopaedic device, for example a plate, is to be fastened. The closed plug indicated by reference numeral 33 is placed in the hole or opening 31 in the bone and a hole or slot in the orthopaedic device 34 to be fixed to the bone is placed above it and the retention means, in this case a screw 35 is introduced through the opening 36 in the orthopaedic device and into the end of the bore 27 of the previously placed plug.

As mentioned above this retention element is either a screw or circumferentially ribbed pin. This is introduced into the cavity in the plug by either screwing, hammering or a combination of these. On insertion the screw or pin , which is generally cylindrical with an overlying tooth form engages in the internal bore of the plug and due to the conical overall shape of the internal bore 13 causes the segments of the plug to be displaced into the bone causing a tight interlocking. Thus the various fins on the outer surface of the segments have a depth to allow significant interlocking with cancellous bone. When a grip is required in less compliant bone such as cortical bone then alternative smaller external engaging surface forms and a lower degree of expansion of the fastener can be used. In the arrangement described the external tooth form of the various fins is such that when engaging bone these do not allow for any rotational unlocking of the fastener.

The advantages of the device are that preparation is simple merely by drilling a hole into bone and the use of a two part construction, that is a plug and separate retention means allows the materials to be better utilised, that is the retention means, in the form of a pin or screw acts as a spacer and requires high tensile strength, this is obviously compatible with a metal but also with an aligned or directionally fibre reinforced polymer matrix material system, which can be relatively easily manufactured into an essentially simple cylindrical structure.

A third advantage is that the plug is a spacing and shear load element with lower specific loading. This is therefor amenable to manufacture in non-reinforced polymers, by injection or compression moulding to form the complex shape required.

The sleeve 17 for preventing expansion of the plug adjacent the open end of the bore can be made from any convenient material, for example metal or a synthetic plastics material.

The particular configuration of the plug blank has numerous advantages as it can be made as a single moulding and folded into its operative position when required. A number of those plugs can also be moulded simultaneously as will be apparent to the person skilled in the art of moulding.

## Claims

1. A fastener for securing an orthopaedic device to a bone comprising an expandable plug made from a biodegradable or biostable material and having two segments which when placed together provide an open ended tapered inner bore provided with retention means, and a retention element which can act on the orthopaedic device to locate it against said bone and which when entered into the bore under pressure locates in the retention means and acts to expand the plug and interconnection means spaced away from the open end of the inner bore for holding said segments in alignment during insertion.

2. A fastener for securing an orthopaedic device to a bone as claimed in claim 1 in which said interconnection between the segments is in the form of a hinge.

3. A fastener for securing an orthopaedic device to a bone as claimed in claim 1 or claim 2 in which said segments have outwardly projecting abutments to engage and locate in an opening in said bone to which the device is to be fastened.

4. A fastener for securing an orthopaedic device to a bone as claimed in claim 3 in which the outwardly projecting abutments have an external tooth form of relatively large pitch.

5. A fastener for securing an orthopaedic device to a bone as claimed in claim 4 in which the abutments include radial and helical fins.

6. A fastener for securing an orthopaedic device to a bone as claimed in claim 3 in which alternative helical fins are opposite handed.

7. A fastener for securing an orthopaedic device to a bone as claimed in claims 1 to 6 in which the retention means in the bore are provided by a series of engagement ribs.

8. A fastener for securing an orthopaedic device to a bone as claimed in claim 6 in which the engagement ribs are in the form of a tapered screw thread.

9. A fastener for securing an orthopaedic device to a bone as claimed in any one of claims 1 to 8 in which the retention element is a screw or a circumferentially ribbed pin.

10. A fastener for securing an orthopaedic device to a bone as claimed in claim 8 in which the screw or pin is of generally cylindrical shape with an overlying tapering tooth form or ribbing.

11. A fastener for securing an orthopaedic device to a bone as claimed in claims 1 to 10 including means for preventing expansion of the plug adjacent the open end of the bore.

12. A fastener for securing an orthopaedic device to a bone as claimed in claim 11 in which the said means comprise a sleeve surrounding the plug.

13. A fastener for securing an orthopaedic device to a bone as claimed in claims 1 to 12 in which the segments are provided with guides to assist assembly together to form the expandable plug.
